# EUROPEAN PATENT SPECIFICATION

(11) **EP 3 264 087 B1**
(45) Date of publication and mention of the grant of the patent: **22.04.2020**
(21) Application number: 16176473.3
(22) Date of filing: 27.06.2016
(51) Int. Cl.: G01N 33/543, C12Q 1/68

(54) **METHOD AND DEVICE FOR QUANTIFICATION OF TARGET MOLECULES**
VERFAHREN UND VORRICHTUNG ZUR QUANTIFIZIERUNG VON ZIELMOLEKÜLEN
PROCÉDÉ ET DISPOSITIF POUR LA QUANTIFICATION DE MOLÉCULES CIBLES

(43) Date of publication of application: 03.01.2018
(73) Proprietor: Chimera Biotec GmbH, 44227 Dortmund (DE)
(72) Inventor: Adler, Michael, 27607 Langen-Debstedt (DE); Schröder, Hendrik, 59399 Olfen (DE); Spengler, Mark, 28237 Bremen (DE)
(74) Representative: Viering, Jentschura & Partner mbB Patent- und Rechtsanwälte

(56) References cited:
- EP-A2- 2 290 100
- WO-A1-2015/013681
- WO-A2-2011/047087
- US-A1- 2009 258 438
- J HE ET AL: "Immunoliposome-PCR: a generic ultrasensitive quantitative antigen detection system", JOURNAL OF NANOBIOTECHNOLOGY, vol. 10, no. 1, 2012, page 26, XP021133042,
- T KUCZIUS ET AL: "Simultaneous detection of three CNS indicator proteins in complex suspensions using a single immuno-PCR protocol", ANALYTICAL BIOCHEMISTRY, vol. 431, no. 1, 2012, pages 4-10, XP028951542,
- M RUDCHENKO ET AL: "Autonomous molecular cascades for evaluation of cell surfaces", NATURE NANOTECHNOLOGY, vol. 8, no. 8, 2013, pages 580-586, XP055237182,
- C NIEMEYER ET AL: "Detecting antigens by quantitative immuno-PCR", NATURE PROTOCOLS, vol. 2, no. 8, 2007, pages 1918-1930, XP002450432,

## Description

### FIELD OF THE INVENTION

The present invention lies in the field of biochemistry and relates to a method for quantifying a plurality of target molecules in a sample. The present invention also relates to the use of a fusion molecule to dissolve the non-covalent bond of a conjugate, and a kit comprising a detection conjugate, a dissolving reagent and nucleic acid amplification agents.

### BACKGROUND OF THE INVENTION

Molecular methods and diagnostic tests have to have a very high degree of sensitivity and specificity to provide a valuable tool in determining the presence of low concentrated molecules and early onset of disease markers. For an improved analysis of the data, it is advantageous if the method or test allows the (precise) quantification of the molecule of interest. There are a number of molecules present within serum, for example, interleukins and parathyroid hormone related protein, which are potential markers of cancer and other pathological conditions. Currently these molecules are only measurable during the late stages of the disease process when they are overexpressed by tumors. Under normal conditions the proteins are present at concentrations of 0.1 pM. Moreover, the early detection of pathogenic organisms in an infection can be critical to whether or not an infected subject survives the infection. This is particularly the case in diseases such as bacterial meningitis and septicemia caused, for example by *Staphyloccocus aureaus.* The earlier and the more precise these molecules can be measured during the disease process the better the prognosis. However, early detection means that the molecules are at low concentrations and the signaling/quantitation systems of current immunoassays, using enzymes and chemiluminescence does not provide sufficient sensitivity to measure at these low levels or only provide imprecise.

One possibility to detect molecules of interest with high sensitivity is the application of immuno-PCR (IPCR). Here, the detected target molecule is coupled to a conjugate of a binding molecule and marker DNA ("detection conjugate") (preferred are multimeric detection conjugates that comprise several binding molecules and several DNA markers).

The binding molecule (preferably a specific antibody, but alternatively also a receptor or an antigen, if the detected target molecule itself is an antibody) recognizes the target molecule. The DNA is subsequently amplified (preferably in a PCR reaction, but alternatively also in a isothermal amplification reaction; detected by a DNA amplification signal-generating probe, for example a TaqMan probe, which is degraded during the extension of a complementary DNA strand by the exonuclease activity of the Taq-polymerase and generates a fluorescent signal) and thus provides signal generation and signal amplification.

Critical for the quality of the IPCR is the complete separation of specifically bound detection conjugate and non-specific, unbound detection conjugate. The presence of unspecific bound detection conjugate generates a background signal. To reduce the background signal, the immuno-PCR is preferably carried out in a surface-bound sandwich method.

Here, a capture binding molecule is immobilized on a surface, which binds the target molecule and - at the same time ("combined") or in two separate steps ("sequential") - the detection conjugate. Before addition of the signal generating reagents (e.g. PCR agents and probe), a stringent wash step is carried out which removes non-specifically bound detection conjugate. Additional suitable washing and blocking steps to minimize non-specific interactions can be applied subsequently to the respective incubation steps. In addition, the target molecule can be diluted with a suitable buffer to minimize matrix effects (e.g. of biological sample material).

This IPCR method provides a sensitive method to detect a molecule of interest. However, by using IPCR it is difficult, time consuming, expensive and imprecise to quantify the amount of the molecules of interest present in the original sample. Hence, there is need in the art for a method that allows the sensitive and precise quantification of a molecule of interest.

T. Kuczius et al, Analytical Biochemistry 431 (2012) 4-10 discloses the simultaneous detection of three CNS indicator proteins in complex suspensions using a single immuno-PCR protocol.

### SUMMARY OF THE INVENTION

It is an object of the present invention to meet the above need by providing a method for quantifying a plurality of target molecules as described herein. Surprisingly, the present inventors found that the advantages of immuno-PCR (IPCR), such as high sensitivity, can be connected with the advantages of digital-PCR, such as precise quantification, by coupling both of the above-mentioned methods. In this context, the whole surface-bound detection conjugate of an IPCR or the part representing the nucleic acid marker of the digital-PCR has to be eluted from the surface for further processing. Surprisingly, the present inventors have found that the elution of a plurality of nucleic acid markers can be efficiently achieved by adding a release agent dissolving a (non-covalent) binding between the nucleic acid marker and the other components of the detection conjugate. Thereby, a plurality of nucleic acid markers is eluted from the surface-bound detection conjugate:target molecule complex and can be used in a plurality of parallel amplification reactions to quantify of the amount of target molecules present in the original sample.

In a first aspect, the present invention is thus directed to a method for quantifying a plurality of target molecules in a sample, comprising a) providing (i) the plurality of target molecules, wherein the target molecules are immobilized on a solid substrate, and (ii) a plurality of detection conjugates each comprising a binding molecule binding specifically to the target molecule and a nucleic acid marker, wherein the binding molecule and the nucleic acid marker are non-covalently linked, b) contacting the plurality of target molecules and the plurality of detection conjugates under conditions that allow specific binding of the binding molecules to the target molecules to form detection conjugate:target molecule complexes, c) contacting the detection conjugate:target molecule complexes formed in step (b) with (i) a release agent that allows release of the nucleic acid markers from the plurality of detection conjugate:target molecule complexes, wherein the release is facilitated by competitive binding of the release agent to at least one member of the non-covalently linked detection conjugate, and (ii) optionally nucleic acid amplification agents, d) separating the released nucleic acid markers from the binding molecule:target molecule complexes, e) diluting the released nucleic acid markers to obtain a plurality of amplification mixtures that are spatially separated, each comprising amplification agents and none or at least one nucleic acid marker molecule, f) subjecting the plurality of amplification mixtures of step e) to conditions that allow amplification of the nucleic acid marker, g) detecting the presence or absence of a signal indicating the amplification of the nucleic acid marker in each of the plurality of amplification mixtures individually and determining the amount of amplification mixtures having a positive signal, and h) comparing the amount determined in step g) with a reference, thereby quantifying the amount of the target molecule in the sample.

In various embodiments of the invention, the target molecule is a) directly attached to the solid substrate, or b) attached to the solid substrate by being bound to a capture molecule which is attached to the solid substrate. In other various embodiments, the method comprises a separation step between step b) and c) to remove unbound detection conjugate. In still other various embodiments, the detection of the amplification of the nucleic acid marker in step (g) comprises detection by a detection probe.

The scope of the present invention also encompasses various embodiments wherein the non-covalent binding between the binding molecule and the nucleic acid marker of the detection conjugate is facilitated by streptavidin/biotin or avidin/biotin interaction. In other various embodiments, the release agents comprise a nucleic acid primer that is covalently linked to the release agent.

In still further various embodiments of the invention, the amplification is a) a PCR reaction, or b) an isothermal reaction, optionally selected from the group consisting of nucleic acid sequence-based amplification (NASBA), transcription mediated amplification (TMA), Loop-mediated Isothermal Amplification (LAMP), Helicase-Dependent isothermal Amplification (HDA), Recombinase Polymerase Amplification (RPA) and strand displacement amplification (SDA). In other various embodiments, the detection conjugate comprises or consists of a biotinylated antibody, a tetravalent biotin-binding streptavidin (STV) and a biotinylated nucleic acid marker.

Also encompassed by the scope of the present invention is that in various embodiments the target molecule is an antibody that is attached to the solid substrate by the interaction with an antigen immobilized on the solid substrate and wherein the binding molecule of the detection conjugate is also an antigen of said antibody.

In various embodiments, the preparation of the plurality of amplification mixtures is carried out by using a) droplets/vesicles for encapsulation of the amplification mixture; or b) microcavities.

In further various embodiments of the invention, the non-covalent bond between the binding molecule and the nucleic acid is formed *in situ* at the target molecule.

In a further aspect, the present invention relates to the use of a fusion molecule comprising a nucleic acid primer and a release agent to dissolve the non-covalent bond of a conjugate comprising a nucleic acid molecule and a second molecule, wherein the release agent dissolves the non-covalent bond by competitive binding to at least one member of the non-covalent bond and wherein the non-covalent binding between the nucleic acid molecule and the second molecule is formed by streptavidin/biotin or avidin/biotin interaction.

In a still further aspect of the invention, the scope encompasses a kit comprising a) a detection conjugate comprising a binding molecule binding specifically to a given target molecule and a nucleic acid marker, wherein the binding molecule and the nucleic acid marker are linked by non-covalent binding, b) a dissolving reagent that dissolves the non-covalent bond between the binding molecule and the nucleic acid marker of the detection conjugate by competitive binding to at least one member of the non-covalent bond, and c) nucleic acid amplification agents, wherein the amplification agents of the kit comprise a nucleic acid primer that is covalently attached to the dissolving agent.

### BRIEF DESCRIPTION OF THE DRAWINGS

The invention will be better understood with reference to the detailed description when considered in conjunction with the non-limiting examples and the accompanying drawings.

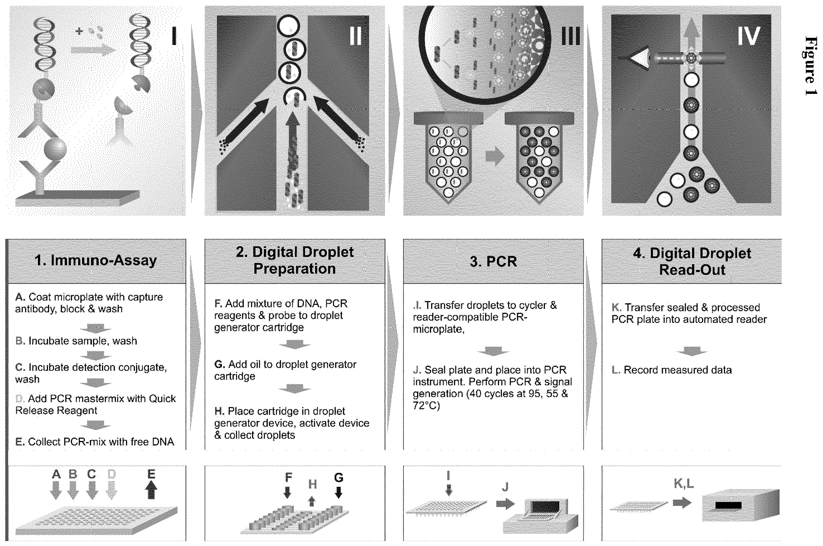

**Figure 1** shows a schematic diagram of a digital immuno-PCR (IPCR) exemplified by a digital droplet IPCR.

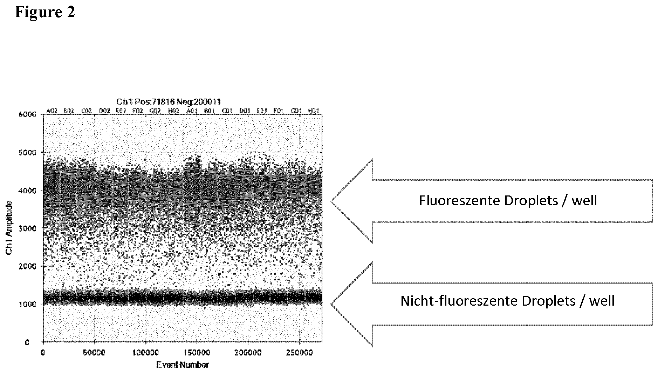

**Figure 2** shows the data of a digital droplet ("DD") IPCR of interleukin 6 (IL-6).

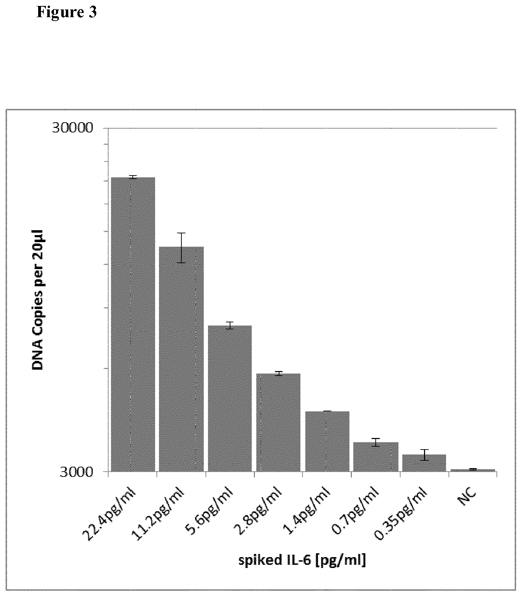

**Figure 3** shows the correlation of a IL-6 dilution series and the amount of DNA marker copies, which was determined by digital droplet analysis (mean of duplicates).

### DETAILED DESCRIPTION OF THE INVENTION

The present inventors surprisingly found that immune-PCR technology and digital-PCR technology can be combined to establish a sensitive and precise method for quantification of target molecules of interest. To combine both technologies, an elution step is required that separates a nucleic acid marker from a detection conjugate/target molecule complex, which is immobilized on a solid substrate. This elution may be achieved by enzymatic or chemical cleavage. However, these reactions require adding of further agents that may interfere with subsequent steps of the method. Alternatively, the nucleic acid marker and the detection conjugate may be cleaved by thermic denaturation. Nonetheless, such reaction is complicated and requires the handling of a hot solution that comprises the risk of contamination (heat generated aerosols may affect contamination).

The present inventors surprisingly found that a (non-covalent) bond between the nucleic acid marker and the detection conjugate can be dissolved by a release agent that (competitively) binds to at least one member of the detection conjugate. The application of the release agent results in effective and constant elution of the nucleic acid marker, which can be used in subsequent reaction for amplification and quantification. For example, by using a non-covalent bond between the nucleic acid marker and the detection conjugate and by dissolving said bond with a competitive binding agent, the above-described disadvantages are overcome.

Therefore, in a first aspect, the present invention is thus directed to a method for quantifying a plurality of target molecules in a sample, comprising a) providing (i) the plurality of target molecules, wherein the target molecules are immobilized on a solid substrate, and (ii) a plurality of detection conjugates each comprising a binding molecule binding specifically to the target molecule and a nucleic acid marker, wherein the binding molecule and the nucleic acid marker are non-covalently linked, b) contacting the plurality of target molecules and the plurality of detection conjugates under conditions that allow specific binding of the binding molecules to the target molecules to form detection conjugate:target molecule complexes, c) contacting the detection conjugate:target molecule complexes formed in step (b) with (i) a release agent that allows release of the nucleic acid markers from the plurality of detection conjugate:target molecule complexes, wherein the release is facilitated by competitive binding of the release agent to at least one member of the non-covalently linked detection conjugate, and (ii) optionally nucleic acid amplification agents, d) separating the released nucleic acid markers from the binding molecule:target molecule complexes, e) diluting the released nucleic acid markers to obtain a plurality of amplification mixtures that are spatially separated, each comprising amplification agents and none or at least one nucleic acid marker molecule, f) subjecting the plurality of amplification mixtures of step e) to conditions that allow amplification of the nucleic acid marker, g) detecting the presence or absence of a signal indicating the amplification of the nucleic acid marker in each of the plurality of amplification mixtures individually and determining the amount of amplification mixtures having a positive signal, and h) comparing the amount determined in step g) with a reference, thereby quantifying the amount of the target molecule in the sample.

The terms "quantifying", and "quantification", which are used interchangeably herein, refer to processes that relate to or involve the measurement of quantity or the amount of the target molecule or signal of corresponding to the amount of target molecule in a sample (also referred as quantitation), which includes but is not limited to any analysis designed to determine the amounts or proportions of the target molecule or its signal. The quantitatively detection refers to a detection method which provides results describing the amount and type of the target molecule. The quantity can be either an absolute number of copies of the target molecules or a relative amount normalized to a standard amount of the target molecule (for the example, the average amount of the target molecule found in a group of healthy individuals or in a group of specific patients) or normalized to a reference molecule, such as the gene product of a housekeeping gene.

The term "one or more", as used herein, in connection with molecules relates to at least one, but preferably at least 2, 3, 4, 5, 6, 7, 8, 9, 10, 12, 14, 15, 20, 25 or a plurality of molecules. In this connection, the term "plurality" means more than one, preferably 2-1000, more preferably 2-100, even more preferably 2-50, still more preferably 2-25 and most preferably 2-15.

The terms "analyte", "target compound", "target molecule" or "target", as interchangeably used herein, refer to any substance that can be detected with the present method assay by binding to a binding molecule, and which may be present in a sample. Therefore, the analyte can be, without limitation, any substance for which there exists a naturally occurring antibody or for which an antibody can be prepared. The analyte may, for example, be an antigen, a protein, a polypeptide, a hapten, a carbohydrate, a lipid, a metabolite, a cell or any other of a wide variety of biological or non-biological molecules, complexes or combinations thereof. Generally, the analyte will be a protein, peptide, carbohydrate or lipid derived from a biological source such as bacterial, fungal, viral, plant or animal samples. Additionally, however, the target may also be a small organic compound such as a drug, drug-metabolite, dye or other small molecule present in the sample.

The term "sample", as used herein, refers to an aliquot of material, frequently biological matrices, an aqueous solution or an aqueous suspension derived from biological material. Samples to be assayed for the presence of an analyte by the methods of the present invention include, for example, cells, tissues, homogenates, lysates, extracts, and purified or partially purified proteins and other biological molecules and mixtures thereof.

Non-limiting examples of samples typically used in the methods of the invention include human and animal body fluids such as whole blood, serum, plasma, cerebrospinal fluid, sputum, bronchial washing, bronchial aspirates, urine, semen, lymph fluids and various external secretions of the respiratory, intestinal and genitourinary tracts, tears, saliva, milk, white blood cells, myelomas and the like; biological fluids such as cell culture supernatants; tissue specimens which may or may not be fixed; and cell specimens which may or may not be fixed. The samples used in the methods of the present invention will vary based on the assay format and the nature of the tissues, cells, extracts or other materials, especially biological materials, to be assayed. Methods for preparing protein extracts from cells or samples are well-known in the art and can be readily adapted in order to obtain a sample that is compatible with the methods of the invention.

The term "solid support" or "solid substrate", as interchangeably used herein, refer to a solid or insoluble substrate/support, commonly a polymeric support, to which a linker moiety (that allows binding of the target molecule or a capture molecule) can be covalently bonded by reaction with a functional group of the support. Many suitable supports are known, and include materials such as polystyrene resins, polystyrene/divinylbenzene copolymers, agarose, and other materials known to the skilled person skilled in the art. It will be understood that an insoluble support can be soluble under certain conditions and insoluble under other conditions; however, for purposes of this invention, a polymeric support is "insoluble" if the support is insoluble or can be made insoluble in a reaction solvent. Further, the solid support may be a soluble or insoluble polymeric structure, such as polystyrene, or an inorganic structure, e.g. of silica or alumina. "Immobilization on the solid substrate" refers to the covalent bond of the target molecule or a capture molecule to said substrate.

The term "detection conjugate" or "detection molecule", as interchangeably used herein, refers to any molecule or target-binding fragment thereof capable of specifically binding to the target molecule so as to form a specific complex consisting of the detection conjugate and the target. In case of the presence of another binding molecule coupling the detection conjugate/target molecule complex to the solid substrate, the detection conjugate is a second binding molecule used for the specific detection of the analyte. In this case, two binding molecules are used for the specific binding of the analyte in a "sandwich" assay. During sandwich assay, the other binding molecule is termed "capture" molecule. In case of the direct immobilization of the target molecule against a surface without a capture molecule, the detection conjugate is the only binding molecule used for the specific binding of the analyte. In general, the detection conjugate comprises two parts: the first part of the detection molecule allows specific binding of the analyte; the second part comprises or consists of a nucleic acid marker. The "binding molecule", as used herein, may be an antibody, an antigen (if the target molecule is an antibody), a small molecule, a receptor, a ligand (if the target molecule is a receptor), an aptamer or a lipocalin.

The term "detection conjugate:target molecule complex", as used herein, refers to a chemical complex formed by the detection conjugate and the target molecule. Said complex is formed when the binding molecule of the detection conjugate binds to the target molecule after both molecules have been brought into contact.

"Specifically binding" and "specific binding", as used herein, means that the binding molecule binds to the target molecule based on recognition of a binding region or epitope on the target molecule. The binding molecule preferably recognizes and binds to the target molecule with a higher binding affinity than it binds to other compounds in the sample. In various embodiments of the invention, "specifically binding" may mean that an antibody or other biological molecule, binds to a target molecule with at least about a 10⁶-fold greater affinity, preferably at least about a 10⁷-fold greater affinity, more preferably at least about a 10⁸-fold greater affinity, and most preferably at least about a 10⁹-fold greater affinity than it binds molecules unrelated to the target molecule. Typically, specific binding refers to affinities in the range of about 10⁶-fold to about 109-fold greater than non-specific binding. In some embodiments, specific binding may be characterized by affinities greater than 10⁹-fold over non-specific binding. In a specific embodiment, the binding molecule uniquely recognizes and binds to the target molecule.

The term "nucleic acid marker" refers to a nucleic acid molecule that will produce a detection product of a predicted size or other selected characteristic when used with appropriately designed oligonucleotide primers in a nucleic acid amplification reaction, such as a PCR reaction. The skilled person is familiar with the design of suitable oligonucleotide primers for PCR and programs are available over the Internet to facilitate this aspect of the invention (cf., for example, the http site: bibiserv.techfak.unibielefeld.de/genefisher). A nucleic acid marker may be linear or circular. In preferred embodiments, the nucleic acid marker will comprise a predetermined, linear nucleic acid sequence with binding sites for selected primers located at or near each end. In a circular DNA nucleic acid molecule, the primers will be internal rather than at an end, and a single primer may be used, e.g. for Rolling Circle Amplification. Amplified DNA may be detected using any available method, including, but not limited to techniques such as real time PCR, SYBR Green staining, or ethidium bromide staining. In other embodiments of the invention, the binding sites for the amplification primers flank an undefined DNA sequence of defined length or which comprises another identifiable characteristic, such as a detectable sequence, in addition to undefined sequences. In some embodiments, the nucleic acid marker are distinguished by the size or mass of the amplified sequences; thus, the DNA sequence between the primers need not be defined as to the exact sequence, just as to the number of bases. Alternatively, the size and/or sequence of the entire nucleic acid marker need not be defined as long as a binding site for a molecular beacon is supplied. In further embodiments, the DNA sequence located between the primer binding sites comprises a "characteristic identification sequence" capable of being detected during the PCR reaction. Fluorescent signal generation may, for example, be sequence-specific (Molecular Beacons, TaqMan, fluorogenic primers, such as the LUX primers (Invitrogen (Carlsbad, CA.)) or mass dependent (SYBR Green, Ethidium Bromide). In preferred embodiments, Molecular Beacons or a TaqMan probe is used to detect the amplification of the nucleic acid marker. Such systems that allow the detection of amplification by generating a specific (fluorogenic) signal are herein referred to as an "amplification detection probe". The examples provided are not meant to be an exhaustive list of possible nucleic acid detection schemes as those skilled in the art will be aware of alternative markers suitable for use in the methods of the present invention.

The term "non-covalent", as used herein, for example in the context of non-covalent binding of the binding molecule to the nucleic acid marker to form the detection conjugate, refers to a bond between two chemical moieties, which is not formed by covalent binding. Examples of different types of non-covalent bonds include, but are not limited to, ion bonds, hydrogen bonds and bonds due to van der Waals forces, Coloumb forces and/or London forces. The non-covalent bond between the binding molecule and the nucleic acid marker may be established by linking each of the above molecules with one member of a binding pair. However, the non-covalent bond may also be formed by indirect non-covalent interaction of functional groups linked to the binding molecule and the nucleic acid marker. One such example is the case, wherein each of the binding molecule and the nucleic acid marker are biotinylated and the non-covalent bond is established by a multivalent streptavidin or avidin molecule. Further non-limiting examples of binding pairs that allow after coupling non-covalent binding between the binding molecule and the nucleic acid marker are two compounds that specifically bind to one another, such as (functionally): a receptor and a ligand (such as a drug), an antibody and an antigen, etc.; or (structurally): protein or peptide and protein or peptide; protein or peptide and nucleic acid; and nucleotide and nucleotide etc. Non-covalent binding pairs include, but are not limited to antigen-antibody, receptor-hormone, receptor-ligand, agonist-antagonist, lectin-carbohydrate, nucleic acid (RNA or DNA) hybridizing sequences, Fc receptor or mouse IgG-protein A, avidin-biotin, streptavidin-biotin. Non-covalent binding pairs may also include a c-myc tag, an HA-tag, a T7 tag, a FLAG-tag, a polyhistidine tag (such as (His)₆), a polyarginine tag, a polyphenylalanine tag, a polycysteine tag, or a polyaspartic acid tag and the corresponding interaction partner, such as iminodiacetic acid (Ni-IDA), nitrilotriacetic acid (Ni-NTA), carboxylmethylaspartate (Co-CMA) or an antibody.

The term "contacting", as used herein, refers generally to providing access of one component, reagent, analyte or sample to another. For example, contacting can involve mixing a solution comprising the detection conjugate with a sample comprising a target molecule. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The terms "release agent" or "dissolving agent", as used interchangeably herein, refer to an agent that allows the separation of the detection conjugate into the binding molecule and the nucleic acid marker. The detection conjugate contains a non-covalent bond between the binding molecule and the nucleic acid marker. This bond is separated/dissolved by the dissolving agent by non-covalent binding to at least member of the bond. In preferred embodiments, the dissolving agent comprises or consists of a functional group that is identical to at least one functional group that is linked to the binding molecule and/or the nucleic acid marker and forms the non-covalent bond. By providing the dissolving agent in excess over the detection conjugate, the dissolving agent displaces one member of the non-covalent bond by competitive binding. In solution, the excess of the dissolving agent over the amount of detection conjugate may be at least 2-fold, 3-fold, 4-fold, 5-fold, 10-fold, 20-fold, 30-fold, 50-fold, 100-fold, 500-fold or 1000-fold. In alternative embodiments, the dissolving agent comprises or consists of a functional group that has an enhanced affinity to one member of the non-covalent compared to its binding partner linked to the binding molecule and/or the nucleic acid marker. In various embodiments, the functional groups linked to the binding molecule and the nucleic acid marker are not directly binding to each other but their interaction is bridged by a linker molecule. For example, the binding molecule and the nucleic acid marker are each linked to a biotin molecule and their non-covalent binding is formed by a multivalent streptavidin or avidin molecule, which represents the linker molecule. In the above context, the term "competitive binding" refers to the competition between a functional group linked to the binding molecule or the nucleic acid marker and the dissolving agent, called the competitive binding compound, for a limited number of binding sites on a member that forms the non-covalent bond of the detection conjugate. "Members of the non-covalent bond", as used herein, relates to functional groups that are linked to the binding molecule and the nucleic acid marker to establish their interaction. However, as described above, in preferred various embodiments, the functional groups are not binding directly to each other but each binding to a linker molecule. Also such linker molecule, if present, is considered to be a member of the non-covalent bond. Therefore, in case where the binding molecule and the nucleic acid marker are each linked to a biotin molecule and their non-covalent binding is formed by a multivalent streptavidin or avidin molecule, both biotin molecules and the streptavidin or avidin molecule are regarded as members of the non-covalent bond.

"Nucleic acid amplification" or "DNA amplification", as interchangeably used herein, refer to any process that increases the number of copies of a specific DNA sequence by enzymatically amplifying the nucleic acid sequence. A variety of processes are known. One of the most commonly used is the polymerase chain reaction (PCR), which is described in U.S. Pat. Nos. 4,683,195 and 4,683,202. PCR involves the use of a thermostable DNA polymerase, known sequences as primers, and heating cycles, which separate the replicating deoxyribonucleic acid (DNA) strands and exponentially amplify a nucleic acid sequence of interest. Any type of PCR, such as quantitative PCR, RT-PCR, hot start PCR, LAPCR₅ multiplex PCR, touchdown PCR, etc., may be used. In various embodiments, real-time PCR is used, in general, the PCR amplification process involves an enzymatic chain reaction for preparing exponential quantities of a specific nucleic acid sequence. It requires a small amount of a sequence to initiate the chain reaction and oligonucleotide primers that will hybridize to the sequence, in PCR the primers are annealed to denatured nucleic acid followed by extension with an inducing agent (enzyme) and nucleotides. This results in newly synthesized extension products. Since these newly synthesized sequences become templates for the primers, repeated cycles of denaturing, primer annealing, and extension results in exponential accumulation of the specific sequence being amplified. The extension product of the chain reaction will be a discrete nucleic acid duplex with a termini corresponding to the ends of the specific primers employed. Apart from the template nucleic acid strand, which is in the present invention the nucleic acid marker, agents that are required to perform a PCR or an isothermal amplification reaction are "nucleic acid amplification agents". If the nucleic acid marker is amplified by PCR, the nucleic acid amplification agents include, but are not limited to oligonucleotide primer pair, buffer, salts, (DNA) polymerase and deoxynucleoside triphosphate (dNTPs). Such agents are well-known in the art. In various embodiments of the present invention, the amplification agents comprise amplification primers comprising a nucleotide sequence that is at least partly complementary to the nucleotide sequence of the nucleic acid marker.

The term "separating", as used herein, refers to physical separation of two elements (e.g., by size or affinity, etc.) as well as degradation of one element, leaving the other intact.

"Preparing a plurality of amplification mixtures", as used herein, refers to dividing a mixture containing a plurality of eluted nucleic acid markers and nucleic acid amplification agents into a plurality of preparations wherein each preparation comprises nucleic acid amplification agents and none or at least one nucleic acid marker molecule. For the preparation an educated guess or empirical data on the amount of nucleic acid marker can be used to generate amplification mixtures, which contain as the arithmetic mean one nucleic acid marker. Nonetheless, for a plurality of amplification mixtures this means that some mixtures contain none or two or three or even more markers, whereas the majority of mixtures contains one marker. Conditions that allow the amplification of nucleic acids, specifically DNA, independent of the method of amplification (PCR or isothermal amplification reactions) are well-known in the art.

The term "detection", as used herein, relates to quantitatively or qualitatively identification of the amplification of the nucleic acid marker (e.g., DNA or RNA) within the amplification mixture. However, in cases in which the amplification is detected quantitatively, the result is recorded as a binary result: this means that for each mixture of the plurality of amplification mixtures a positive or negative detection is recorded. A positive detection refers to a signal that is significantly enhanced over a comparative background signal. A negative signal refers to a signal that cannot be distinguished from a comparative background signal. The absence or presence of the signal based on the amplification of the nucleic acid marker can be detected by several different techniques known in the art. Such techniques may include, but are not limited to fluorescence assay, mass spectrometry, chromatography, Western Blot, or gel electrophoresis. For example, the amplification of the nucleic acid marker may be detected with signals emitted by a TaqMan probe or a Molecular Beacon. In alternative embodiments, the amplification of the nucleic acid marker is determined by gel electrophoresis and ethidium bromide detection.

"Determining the amount of amplification mixtures having a positive signal", as used herein, may refer to the percentage of amplification mixtures that have a signal that is significantly enhanced over a comparative background signal. The skilled person will understand that for the purpose of the method of the present invention alternatively also the amount of amplification mixtures can be determined that have a negative signal as this is the reciprocal value of the amount of amplification mixtures having a positive signal.

The terms "reference" or "standard curve", as used interchangeably herein, relate to a curve correlating the concentration of a given target molecule in a sample with the amount (e.g. percentage) of positive amplification signals in a plurality of amplification mixtures. Said amplification mixtures are generated from the sample containing the target molecule according to the method steps a) to g) of the present invention.

In various embodiments of the invention, the target molecule is a) directly attached to the solid substrate, or b) attached to the solid substrate by being bound to a capture molecule which is attached to the solid substrate. In other various embodiments, the method comprises a separation step between step b) and c) to remove unbound detection conjugate. In still other various embodiments, the detection of the amplification of the nucleic acid marker in step (g) comprises detection by a detection probe.

The term "directly attached", as used herein, refers to target molecules that are bound to a functional group or functional layer of the solid substrate. Alternatively, the target molecule may be attached to a molecule, which is attached to the solid substrate for the mere purpose of binding the target molecule ("sandwich-assay"). Such molecules are herein referred to as "capture molecules" and include, but are not limited to a monoclonal antibody, a group a polyclonal antibodies, an antigen (if the target molecule is an antibody), a small molecule, a receptor (if the target molecule is a ligand) or a ligand (if the target molecule is a receptor).

The terms "separation step" or "washing step", as interchangeably used herein, refer to a process in which other proteins, lipids, carbohydrates, nucleic acids, and/or other impurities originating from the sample and being not the target molecule are removed from the preparation of the invention. The washing step according to the present invention includes suspension of the target molecule in water, saline and/or lipophilic solution. The solution used for the washing step may be a buffer solution. The washing solution may comprise, for example, a salt, such as NaCl, from about 0.05 to about 0.15 M and/or a lipophilic agent, such as sodium dodecyl sulfate (SLS or SDS), cetyl trimethylammonium bromide (CTAB), octoxinol 9, polysorbate 20, digitonin, dodecyl maltoside, octyl glucoside and surfactants.

The scope of the present invention also encompasses various embodiments wherein the non-covalent binding between the binding molecule and the nucleic acid marker of the detection conjugate is facilitated by streptavidin/biotin or avidin/biotin interaction. In other various embodiments, the release agents comprise a nucleic acid primer that is covalently linked to the release agent.

The terms "polynucleotide" and "nucleic acid (molecule)" are used interchangeably to refer to polymeric forms of nucleotides of any length, including naturally occurring and non-naturally occurring nucleic acids. The polynucleotides may contain deoxyribonucleotides, ribonucleotides and/or their analogs. Methods for selection and preparation of nucleic acids are diverse and well-described in standard biomolecular protocols. A typical way would be preparative PCR and chromatographic purification starting from existing template DNAs or stepwise synthesis of artificial nucleic acids.

Nucleotides may have any three-dimensional structure, and may perform any function, known or unknown. The term "nucleic acid molecule" includes single-, double-stranded and triple helical molecules. "Oligonucleotide" refers to polynucleotides of between 3 and about 100, for example 3-50, 5-30, or 5-20 nucleotides of single- or double-stranded nucleic acid, typically DNA.

The term "nucleic acid molecule" or "nucleic acid sequence", as used herein, relates to DNA (deoxyribonucleic acid) or RNA (ribonucleic acid) molecules. Said molecules may appear independent of their natural genetic context and/or background. The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

Oligonucleotides are also known as oligomers or oligos and may be isolated from genes, or chemically synthesized by methods known in the art. A "primer" refers to an oligonucleotide, usually single-stranded, that provides a 3'-hydroxyl end for the initiation of enzyme-mediated nucleic acid synthesis. In preferred embodiments, the primer and the dissolving agent, for example the portion containing the functional group binding to a member of the non-covalent bond between the binding molecule and the nucleic acid marker, are linked by a covalent bond, such as σ-bonding, π-bonding, metal to metal bonding, agostic interactions, and three-center two-electron bonds.

The following are non-limiting embodiments of nucleic acids: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, nucleic acid marker and primers. A nucleic acid molecule may also comprise modified nucleic acid molecules, such as methylated nucleic acid molecules and nucleic acid molecule analogs. Analogs of purines and pyrimidines are known in the art, and include, but are not limited to, aziridinylcytosine, 4-acetylcytosine, 5-fluorouracil, 5-bromouracil, 5-carboxymethylaminomethyl-2-thiouracil, 5-carboxymethyl-aminomethyluracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, pseudouracil, 5-pentylnyluracil and 2,6-diaminopurine. The use of uracil as a substitute for thymine in a deoxyribonucleic acid is also considered an analogous form of pyrimidine. A nucleic acid may also include a backbone modification, wherein the phosphodiester bonds are replaced with phosphorothioates or methylphosphonates.

In still further various embodiments of the invention, the amplification is a) a PCR reaction, or b) an isothermal reaction, optionally selected from the group consisting of nucleic acid sequence-based amplification (NASBA), transcription mediated amplification (TMA), Loop-mediated Isothermal Amplification (LAMP), Helicase-Dependent isothermal Amplification (HDA), Recombinase Polymerase Amplification (RPA) and strand displacement amplification (SDA). In other various embodiments, the detection conjugate comprises or consists of a biotinylated antibody, a tetravalent biotin-binding streptavidin (STV) and a biotinylated nucleic acid marker.

The term "isothermal amplification", as used herein, indicates a method of DNA amplification using polymerase chain reaction that uses a single temperature incubation thereby obviating the need for a thermal cycler. By combining with a reverse transcription step, these amplification methods can also be used to isothermally amplify RNA. In several embodiments, the methods and apparatus herein described allow isothermal amplification of a nucleic acid marker. For example, in some embodiments, the isothermal amplification of the marker is performed by the Loop-mediated Isothermal Amplification (LAMP). In some embodiments, the isothermal amplification of a target nucleic acid is performed by Helicase-Dependent isothermal Amplification (HDA). In other embodiments, the isothermal amplification of a target nucleic acid is performed by Recombinase Polymerase Amplification (RPA). In other embodiments, the isothermal amplification is selected from the group consisting of nucleic acid sequence-based amplification (NASBA), transcription mediated amplification (TMA) and strand displacement amplification (SDA).

Also encompassed by the scope of the present invention is that in various embodiments the target molecule is an antibody that is attached to the solid substrate by the interaction with an antigen immobilized on the solid substrate and wherein the binding molecule of the detection conjugate is also an antigen of said antibody.

The term "antibody" is used in the broadest sense and specifically covers monoclonal antibodies as well as antibody variants or fragments (e.g., Fab, F(ab')2, scFv, Fv diabodies and linear antibodies), so long as they exhibit the desired binding activity (for a review of scFv see Pluckthun (1994) The Pharmacology of Monoclonal Antibodies, Vol. 113. Rosenburg and Moore eds. Springer-Verlag, New York, pp. 269-315). Diabodies are described more fully in, for example, European patent 404097, international patent publication WO 93/11161 and Hollinger et al. (1993) Proc. Natl. Acad. Sci. USA 90: 6444-6448. Linear antibodies are described in Zapata et al. (1995) Protein Eng. 8(10): 1057-1062.

The term "monoclonal antibody", as used herein, refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they may be synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies can include "chimeric" antibodies (U.S. Patent No. 4,816,567; and Morrison et al. (1984) Proc. Natl. Acad. Sci. USA, 81: 6851-6855) and humanized antibodies (Jones et al. (1986) Nature, 321: 522-525 ; Reichmann et al. (1988) Nature, 332: 323-329 ; Presta (1992) Curr. Op. Struct. Biol. 2: 593-596). A "chimeric" antibody is a molecule in which different portions are derived from different animal species, such as those having a variable region derived from a murine mAb and a human immunoglobulin constant region.

Monoclonal antibodies may be obtained by any technique that provides for the production of antibody molecules by continuous cell lines in culture. These include, but are not limited to the hybridoma technique of Koehler and Milstein (1975), Nature, 256: 495-7 ; and U. S. Patent No. 4,376,110), the human B-cell hybridoma technique (Kosbor, et al. (1983), Immunology Today, 4: 72 ; Cote, et al. (1983), Proc. Natl. Acad. Sci. USA, 80: 2026-30), and the EBV-hybridoma technique (Cole, et al. (1985), in Monoclonal Antibodies And Cancer Therapy, Alan R. Liss, Inc., New York, pp. 77-96). The preparation of monoclonal antibodies specific for a target compound is also described in Harlow and Lane, eds. (1988) Antibodies - A Laboratory Manual. Cold Spring Harbor Laboratory, Chapter 6. Such antibodies may be of any immunoglobulin class including IgG, IgM, IgE, IgA, IgD and any subclass thereof. The hybridoma producing the mAb may be cultivated *in vitro* or *in vivo.* Production of high titers of mAbs *in vivo* makes this a very effective method of production.

"Polyclonal antibodies" are heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

Alternatively, techniques described for the production of single chain antibodies (U. S. Patent No. 4,946,778 ; Bird (1988), Science 242: 423-26 ; Huston, et al. (1988), Proc. Natl. Acad. Sci. USA, 85: 5879-83 ; and Ward, et al. (1989), Nature, 334: 544-46) can be adapted to produce gene-single chain antibodies. Single chain antibodies are typically formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')2 fragments that can be produced by pepsin digestion of the antibody molecule and the Fab fragments that can be generated by reducing the disulfide bridges of the F(ab')2 fragments. Alternatively, Fab expression libraries may be constructed (Huse, et al. (1989), Science, 246: 1275-1281) to allow rapid and easy identification of monoclonal Fab fragments with the desired specificity.

In various embodiments, the preparation of the plurality of amplification mixtures is carried out by using a) droplets/vesicles for encapsulation of the amplification mixture; or b) microcavities.

The formation of droplets/vesicles encapsulating molecules of interest is well-known in the art. Methods describing the formation of droplets are disclosed, for example, by Sharma et al. (Sharma, S. et al. (2012) Microfluidic Diagnostics, volume 949, pages 207-230). Such methods comprise, but are not limited to dielectrophoresis (DEP) and electrowetting on dielectric (EWOD). The term "microcavity", refers to a solid support that contains a plurality of equal cavities, wherein each of such cavities has a volume of at most 1500 µl, preferably 1-1000 µl, more preferably 5-500 µl, even more preferably 10-100 µl, still more preferably 15-50 µl and even still more preferably 20-30 µl. Such solid support comprising the microcavities may be a thin-film (e.g. a polymer) wherein the cavities are formed by contact-patterning using polydimethylsiloxane stamps (see, for example, Scott, B.M. and Bulovic, V. (2012) Photonics Technology Letters, volume 24, pages 104-106). However, the skilled is aware of several alternative methods to produce solid supports comprising microcavities, wherein the solid support may be made of, but not limited to a polymeric, metallic (e.g. gold), glass or hybrid material. The solution containing the nucleic acid marker and the amplification agent can be filled into the microcavities by automated pipetting. The above described droplets and microcavities are used to form a plurality of amplification mixtures. Said plurality of mixtures is prepared from one origin solution. By enlarging or reducing the volume of the amplification mixture each mixture of the plurality only contains one nucleic acid marker (mean value; see also above). The purpose of the droplets or the microvaties is to keep the individual amplification mixtures separate from each other. Therefore, all methods that allow the separation of a plurality of mixtures, such as droplet vaporization, can be used in the method of the present invention.

In further various embodiments of the invention, the non-covalent bond between the binding molecule and the nucleic acid is formed *in situ* at the target molecule.

The term *"in situ"*, as used herein, means at the location of the target molecule attached to the solid substrate.

In a further aspect, the present invention relates to the use of a fusion molecule comprising a nucleic acid primer and a release agent to dissolve the non-covalent bond of a conjugate comprising a nucleic acid molecule and a second molecule, wherein the release agent dissolves the non-covalent bond by competitive binding to at least one member of the non-covalent bond, wherein the non-covalent binding between the nucleic acid molecule and the second molecule is formed by streptavidin/biotin or avidin/biotin interaction.

In a still further aspect of the invention, the scope encompasses a kit comprising a) a detection conjugate comprising a binding molecule binding specifically to a given target molecule and a nucleic acid marker, wherein the binding molecule and the nucleic acid marker are linked by non-covalent binding, b) a dissolving reagent that dissolves the non-covalent bond between the binding molecule and the nucleic acid marker of the detection conjugate by competitive binding to at least one member of the non-covalent bond, and c) nucleic acid amplification agents, wherein the amplification agents of the kit comprise a nucleic acid primer that is covalently attached to the dissolving agent.

The term "kit", as used herein, relates to packaged reagents for quantification of a given target molecule. Accordingly, the kits of the invention comprise a detection conjugate, a dissolving agent and nucleic acid amplification agents. Additionally, such a kit may comprise instructions for use as well as typical reagents known to those skilled in the art.

Further disclosed is a device to perform the method of the invention, comprising a) a unit that allows droplet/vesicle preparation, and b) a unit that allows nucleic acid amplification.

A "device", as used herein, refers to an apparatus that can perform all or some steps of the method of the invention. A unit that allows droplet/vesicle preparation refers to one portion of the device in which droplets can be formed containing the nucleic acid marker and the nucleic acid amplification agent. This unit uses the above describes technologies for the preparation of droplets. A unit that allows nucleic acid amplification refers to another portion of the device which amplifies the nucleic acid marker according to at least one method as described above. In preferred embodiments, the two units of the device of the invention are interconnected to transfer the droplet containing solution to the amplification unit. Compared to two devices containing the above described units separately, the device of the present invention provides the advantage of direct and automatic transfer of the droplet solution resulting in more efficiency (less pipetting work) and a minimized risk of contamination. In even more preferred embodiments, the device of the invention comprises a third unit allowing the measurement of the amplification signal. This third unit may be interconnected with the amplification unit to allow the direct and automatic transfer of the amplification mixture.

The term "fusion protein", as used herein, generally indicates a polypeptide in which heterogenous polypeptides having different origins are linked, and in the present invention, can refer to a binding molecule or a nucleic acid marker which are linked to functional group, such as biotin, allowing the formation of a non-covalent bond.

The term "sequence", as used herein, relates to the primary nucleotide sequence of nucleic acid molecules or the primary amino acid sequence of a protein.

The term "conjugate", as used herein, refers to a compound comprising two or more molecules (e.g., peptides, carbohydrates, small molecules, or nucleic acid molecules) that are chemically linked. The two or molecules desirably are chemically linked using any suitable chemical bond (e.g., non-covalent or covalent bond). Suitable chemical bonds are well-known in the art and include hydrophobic bonds, electrostatic bonds, hydrogen bond, disulfide bonds, acid labile bonds, photolabile bonds, peptidase labile bonds (e.g. peptide bonds), thioether, and esterase labile bonds.

The term "linker" or "linker molecule" refers to a molecule that interconnects two or more functional groups or molecules. The linker molecules according to the present invention are chemically distinct from the nucleic acid marker and the binding molecule and are capable of binding the binding molecule and the nucleic acid marker and/or other, chemically different linker molecules. To achieve formation of a detection conjugate complex according to the invention, the linker molecules of the invention are at least bivalent, preferably trivalent, tetravalent, pentavalent, hexavalent or multivalent. In this connection, the term "multivalent" relates to linker molecules that can bind more than 2, preferably more than 3 other molecules. The multiple molecules bound by the linker molecules may be the same or different. For example, a linker molecule may have binding sites for the nucleic acid marker, the binding molecule and/or another, chemically different linker molecules or, alternatively, 2, 3, 4 or more binding sites for one specific binding partner. In the latter case, complex formation is achieved by coupling one or more binding partner(s) to other components of the conjugate complex, such as the nucleic acid marker, the binding molecule and another, chemically different linker molecules. In this connection, the expression "binding partner" relates to a molecule which is specifically recognized and bound by a linker molecule. The binding partner may thus be a small organic molecule, but can also be any other molecule, such as, for example, a peptide, polypeptide, protein, saccharide, polysaccharide or a lipid or an antigen or hapten. Specific examples for such a pair of linker molecule and binding partner are the streptavidin/biotin and avidin/biotin binding pairs. If the linker molecule is streptavidin/avidin and the binding partner is biotin, the biotin may be coupled to either one or all of the binding molecule, the nucleic acid marker and the second linker molecule to facilitate conjugate complex formation. The binding of the linker molecule to its binding partner and/or the nucleic acid marker, the binding molecule and/or other, chemically distinct linker molecules is non-covalent. The linker molecules according to the invention may comprise one or more molecules selected from the group consisting of polysaccharides, organic polymers, polypeptides and nucleic acids distinct from the nucleic acid marker. In case the linker molecule according to the invention comprises a nucleic acid distinct from the nucleic acid marker, the linker molecule may further comprise a polysaccharide, organic polymer or polypeptide chemically coupled to the nucleic acid part.

The present method links the surface-bound IPCR technology and the digital PCR technology requiring a homogeneous liquid phase. The most convenient solution to put the combination of both technologies into practice is the linkage of the IPCR components to small beads, such as microbeads. However, the use of these beads is not compatible with the use of appropriate readout instruments because the micro cannulas and channels of the readout instrument will be clogged with the microparticles.

So a detachment of the nucleic acid marker from the surface is required. For this purpose, three different solutions are possible:

(I) Enzymatic or chemical cleavage of the detection conjugate. The advantage of this method is a precisely controlled reaction (for instance by restriction enzymes and/or proteases). However, the reagents used for cleavage potentially interfere with DNA amplification and thus require an additional and complicated cleaning step.

(II) Thermal denaturation of the detection conjugate. This solution requires no additional reagents. Nonetheless, the handling of hot DNA solution comprises the risk of contamination due to aerosols formed from the hot solution.

(III) Use of a supramolecular detection conjugate that is resolved by the addition of a competing supramolecular binding partner and thereby releases the nucleic acid marker. The advantages of this method are described above in detail. In a nutshell, this method provides a quick, customized, interference-free and mild release of the nucleic acid marker.

### EXAMPLES

### Example: Detection of interleukin 6 (IL-6) by means of a digital droplet immuno-PCR assay ("DD-IPCR")

Anti-IL-6 capture antibody was immobilized on the surface of a microwell plate (5 µg/ml, 30 µl/well/overnight). The coated surface was then washed, blocked against non-specific interactions, washed again and incubated with a serial dilution series of IL-6 in buffer (22.4 - 0.35 pg/ml, 30 µl/well, 45 min/RT). For source of reagents and all standard wash and blocking steps, see the manufacturer's instructions of Imperacer Workstation / Imperacer Assay Development KitChimera Biotec; http://www.chimera-biotec.com/technology/literature/ (Chimera Biotec GmbH, Dortmund, Germany).

After a further washing step, a detection conjugate comprising previously biotinylated anti-IL-6 antibody, biotin binding tetravalent streptavidin (STV) and biotinylated DNA was added (30 µl/well). The conjugate was incubated for 45 min RT, the wells were washed again and subsequently 30 µl PCR master/well was added. Additionally, the conjugate comprises a DNA-marker specific TaqMan probe and a biotinylated oligonucleotide. After a short incubation (15 min), the liquid was removed from the wells and transferred into a DG8 Cartridge (Bio-Rad, Hercules, California, U.S.A.). The cartridge was charged additionally with Droplet Generation Oil (Bio-Rad, Hercules, California, U.S.A.) and by using a QX200 Droplet Generator (Bio-Rad, Hercules, California, U.S.A.) microvesicles were produced according to the manufacturer's protocol. For a detailed description of the procedure see: http://www.bio-rad.com/de-de/applications-technologies/droplet-digital-pcr-ddpcr-technology.

The droplets were transferred in a PCR compatible microtiter plate, which was sealed, and a standard PCR (according to the protocol of Chimera Biotec GmbH, Dortmund, Germany) was performed in a PCR cycler (Bio-Rad, Hercules, California, U.S.A.).

After completion of PCR, the solution of the PCR plate was transferred and measured in a QX200 Droplet Reader (Bio-Rad, Hercules, California, U.S.A.). By determining the number of droplets having positive signals vs. the total number of droplets per well, the number of DNA markers before PCR was determined by using the software of the device. The number of DNA markers before PCR was correlated with the interleukin amount used in the IL-6 dilution series to use this as a standard curve to quantify, for example, an unknown amount of IL-6 in a sample.

## Claims

1. Method for quantifying a plurality of target molecules in a sample, comprising
a) providing
(i) the plurality of target molecules, wherein the target molecules are immobilized on a solid substrate, and
(ii) a plurality of detection conjugates each comprising a binding molecule binding specifically to the target molecule and a nucleic acid marker, wherein the binding molecule and the nucleic acid marker are non-covalently linked,
b) contacting the plurality of target molecules and the plurality of detection conjugates under conditions that allow specific binding of the binding molecules to the target molecules to form detection conjugate:target molecule complexes,
c) contacting the detection conjugate:target molecule complexes formed in step (b) with
(i) a release agent that allows release of the nucleic acid markers from the plurality of detection conjugate:target molecule complexes, wherein the release is facilitated by competitive binding of the release agent to at least one member of the non-covalently linked detection conjugate, and
(ii) optionally nucleic acid amplification agents,
d) separating the released nucleic acid markers from the binding molecule:target molecule complexes,
e) diluting the released nucleic acid markers to obtain a plurality of amplification mixtures that are spatially separated, each comprising amplification agents and none or at least one nucleic acid marker molecule,
f) subjecting the plurality of amplification mixtures of step e) to conditions that allow amplification of the nucleic acid marker,
g) detecting the presence or absence of a signal indicating the amplification of the nucleic acid marker in each of the plurality of amplification mixtures individually and determining the amount of amplification mixtures having a positive signal, and
h) comparing the amount determined in step g) with a reference, thereby quantifying the amount of the target molecule in the sample.

2. The method of claim 1, wherein the target molecule is
a) directly attached to the solid substrate, or
b) attached to the solid substrate by binding to a capture molecule which is attached to the solid substrate.

3. The method of claim 1 or 2, wherein
a) the method comprises a separation step between step b) and c) to remove unbound detection conjugate, and/or
b) the detection of the amplification of the nucleic acid marker in step (g) comprises detection by a detection probe.

4. The method of any one of claims 1-3, wherein the non-covalent binding between the binding molecule and the nucleic acid marker of the detection conjugate is facilitated by streptavidin/biotin or avidin/biotin interaction.

5. The method of any one of claims 1-4, wherein the release agents comprise a nucleic acid primer that is covalently linked to the release agent.

6. The method of any one of claims 1-5, wherein the amplification is
a) a PCR reaction, or
b) an isothermal reaction, optionally selected from the group consisting of nucleic acid sequence-based amplification (NASBA), transcription mediated amplification (TMA), Loop-mediated Isothermal Amplification (LAMP), Helicase-Dependent isothermal Amplification (HDA), Recombinase Polymerase Amplification (RPA) and strand displacement amplification (SDA).

7. The method of any one of claims 1-6, wherein the detection conjugate comprises or consists of a biotinylated antibody, a tetravalent biotin-binding streptavidin (STV) and a biotinylated nucleic acid marker.

8. The method of any one of claims 1-7, wherein the target molecule is an antibody that is attached to the solid substrate by the interaction with an antigen immobilized on the solid substrate and wherein the binding molecule of the detection conjugate is also an antigen of said antibody.

9. The method of any one of claims 1-8, wherein the preparation of the plurality of amplification mixtures is carried out by using
a) droplets/vesicles for encapsulation of the amplification mixture; or
b) microcavities.

10. The method of any one of claims 1-9, wherein the non-covalent bond between the binding molecule and the nucleic acid is formed *in situ* at the target molecule.

11. Use of a fusion molecule comprising a nucleic acid primer and a release agent to dissolve the non-covalent bond of a conjugate comprising a nucleic acid molecule and a second molecule, wherein the release agent dissolves the non-covalent bond by competitive binding to at least one member of the non-covalent bond, wherein the non-covalent binding between the nucleic acid molecule and the second molecule is formed by streptavidin/biotin or avidin/biotin interaction.

12. A kit comprising
a) a detection conjugate comprising a binding molecule binding specifically to a given target molecule and a nucleic acid marker, wherein the binding molecule and the nucleic acid marker are linked by non-covalent binding,
b) a dissolving reagent that dissolves the non-covalent bond between the binding molecule and the nucleic acid marker of the detection conjugate by competitive binding to at least one member of the non-covalent bond, and
c) nucleic acid amplification agents, wherein the amplification agents comprise a nucleic acid primer that is covalently attached to the dissolving agent.

## Patentansprüche

1. Verfahren zur Quantifizierung einer Vielzahl von Zielmolekülen in einer Probe, umfassend
a) Bereitstellen
(i) der Vielzahl von Zielmolekülen, wobei die Zielmoleküle auf einem festen Substrat immobilisiert sind, und
(ii) einer Vielzahl von Nachweiskonjugaten, die jeweils ein Bindungsmolekül umfassen, das spezifisch an das Zielmolekül bindet und einen Nukleinsäuremarker, wobei das Bindungsmolekül und der Nukleinsäuremarker nicht-kovalent verknüpft sind,
b) In-Kontakt-bringen der Vielzahl von Zielmolekülen und der Vielzahl von Nachweiskonjugaten unter Bedingungen, die das spezifische Binden der Bindungsmoleküle an die Zielmoleküle erlauben, um Nachweiskonjugat:Zielmolekül-Komplexe zu bilden,
c) In-Kontakt-bringen der in Schritt (b) gebildeten Nachweiskonjugat:Zielmolekül-Komplexe mit
(i) einem Freisetzungsmittel, das die Freisetzung der Nukleinsäuremarker aus der Vielzahl von Nachweiskonjugat:Zielmolekül-Komplexen erlaubt, wobei die Freisetzung durch kompetitive Bindung des Freisetzungsmittels an mindestens ein Mitglied des nicht-kovalent gebundenen Nachweiskonjugats erleichtert wird, und
(ii) gegebenenfalls Nukleinsäureamplifikationsmitteln,
d) Trennen der freigesetzten Nukleinsäuremarker von den Bindungsmolekül:Zielmolekül-Komplexen,
e) Verdünnen der freigesetzten Nukleinsäuremarker, um eine Vielzahl von Amplifikationsmischungen zu erhalten, die räumlich getrennt sind, wobei jede Amplifikationsmittel und kein oder mindestens ein Nukleinsäuremarkermolekül umfasst,
f) die Vielzahl der Amplifikationsmischungen des Schrittes e) Bedingungen aussetzen, die die Amplifikation des Nukleinsäuremarkers erlauben,
g) Nachweisen des Vorliegens oder Nichtvorliegens eines Signals, das die Amplifikation des Nukleinsäuremarkers in jeder der Vielzahl von Amplifikationsmischungen einzeln anzeigt und Bestimmen der Menge von Amplifikationsmischungen, die ein positives Signal aufweisen, und
h) Vergleichen der in Schritt g) bestimmten Menge mit einer Referenz, wodurch die Menge des Zielmoleküls in der Probe quantifiziert wird.

2. Das Verfahren nach Anspruch 1, wobei das Zielmolekül
a) direkt an das feste Substrat gebunden ist, oder
b) an das feste Substrat gebunden ist, indem es an ein Fängermolekül gebunden ist, das an das feste Substrat gebunden ist.

3. Das Verfahren nach Anspruch 1 oder 2, wobei
a) das Verfahren einen Trennungsschritt zwischen Schritt b) und c) umfasst, um ungebundenes Nachweiskonjugat zu entfernen, und/oder
b) der Nachweis der Amplifikation des Nukleinsäuremarkers in Schritt g) den Nachweis durch eine Nachweissonde umfasst.

4. Das Verfahren nach einem der Ansprüche 1-3, wobei die nicht-kovalente Bindung zwischen dem Bindungsmolekül und dem Nukleinsäuremarkerdes Nachweiskonjugats durch Streptavidin/Biotin- oder Avidin/Biotin-Wechselwirkung erleichtert wird.

5. Das Verfahren nach einem der Ansprüche 1-4, wobei die Freisetzungsmittel einen Nukleinsäureprimer umfassen, der kovalent an das Freisetzungsmittel gebunden ist.

6. Das Verfahren nach einem der Ansprüche 1-5, wobei die Amplifikation
a) eine PCR Reaktion, oder
b) eine isotherme Reaktion, gegebenenfalls ausgewählt aus der Gruppe bestehend aus Nukleinsäuresequenz-basierter Amplifikation (NASBA), transkriptionsvermittelnder Amplifikation (TMA), Loop-vermittelnder isothermer Amplifikation (LAMP), Helikase-abhängiger isothermer Amplifikation (HDA), Recombinase-Polymerase-Amplifikation (RPA) und Strangverdrängungssamplifikation (SDA).

7. Das Verfahren nach einem der Ansprüche 1-6, wobei das Nachweiskonjugat einen biotinylierten Antikörper, ein tetravalentes, biotinbindendes Streptavidin (STV) und einen biotinylierten Nukleinsäuremarker umfasst oder daraus besteht.

8. Das Verfahren nach einem der Ansprüche 1-7, wobei das Zielmolekül ein Antikörper ist, der an das feste Substrat durch die Wechselwirkung mit einem an das feste Substrat immobilisierten Antigen gebunden ist und wobei das Bindungsmolekül des Nachweiskonjugats auch ein Antigen des Antikörpers ist.

9. Das Verfahren nach einem der Ansprüche 1-8, wobei die Herstellung der Vielzahl von Amplifikationsmischungen durchgeführt wird durch Verwendung
a) von Tröpfchen/Vesikeln zum Einkapseln der Amplifikationsmischung; oder
b) von Mikrokavitäten.

10. Das Verfahren nach einem der Ansprüche 1-9, wobei die nicht-kovalente Bindung zwischen dem Bindungsmolekül und der Nukleinsäure *in situ* am Zielmolekül gebildet wird.

11. Verwendung eines Fusionsmoleküls, umfassend einen Nukleinsäureprimer und ein Freisetzungsmittel, um die nicht-kovalente Bindung eines Konjugats, das ein Nukleinsäuremolekül und ein zweites Molekül umfasst, zu lösen, wobei das Freisetzungsmittel die nicht-kovalente Bindung durch kompetitives Binden an mindestens ein Mitglied der nicht-kovalenten Bindung löst, wobei die nicht-kovalente Bindung zwischen dem Nukleinsäuremolekül und dem zweiten Molekül durch Streptavidin/Biotin- oder Avidin/Biotin-Wechselwirkung gebildet wird.

12. Ein Kit, umfassend
a) ein Nachweiskonjugat, das ein Bindungsmolekül, das spezifisch an ein gegebenes Zielmolekül bindet, und einen Nukleinsäuremarker umfasst, wobei das Bindungsmolekül und der Nukleinsäuremarker durch nicht-kovalente Bindung verbunden sind,
b) ein Lösungsreagens, das die nicht-kovalente Bindung zwischen dem Bindungsmolekül und dem Nukleinsäuremarker des Nachweiskonjugats durch kompetitives Binden an mindestens ein Mitglied der nicht-kovalenten Bindung löst, und
c) Nukleinsäureamplifikationsmittel, wobei die Amplifikationsmittel einen Nukleinsäureprimer umfassen, der kovalent an das Lösungsmittel gebunden ist.

## Revendications

1. Procédé pour quantifier une pluralité de molécules cibles dans un échantillon, comprenant :
a) la mise à disposition de
(i) la pluralité de molécules cibles, où les molécules cibles sont immobilisées sur un substrat solide, et
(ii) une pluralité de conjugués de détection comprenant chacun une molécule de liaison qui se lie spécifiquement à la molécule cible et un marqueur d'acide nucléique, où la molécule de liaison et le marqueur d'acide nucléique sont liés de façon non covalente,
b) la mise en contact de la pluralité de molécules cibles et de la pluralité de conjugués de détection dans des conditions qui permettent une liaison spécifique des molécules de liaison aux molécules cibles afin de former des complexes conjugué de détection : molécule cible,
c) la mise en contact des complexes conjugué de détection : molécule cible formés à l'étape (b) avec
(i) un agent de libération qui permet la libération des marqueurs d'acide nucléique à partir de la pluralité de complexes conjugué de détection : molécule cible, où la libération est facilitée par la liaison compétitive de l'agent de libération à au moins un membre du conjugué de détection lié de façon non covalente, et
(ii) facultativement des agents d'amplification d'acide nucléique,
d) la séparation des marqueurs d'acide nucléique libérés des complexes molécule de liaison : molécule cible,
e) la dilution des marqueurs d'acide nucléique libérés afin d'obtenir une pluralité de mélanges d'amplification qui sont séparés spatialement, chacun comprenant des agents d'amplification et aucune ou au moins une molécule de marqueur d'acide nucléique,
f) le fait de soumettre la pluralité de mélanges d'amplification de l'étape e) à des conditions qui permettent une amplification du marqueur d'acide nucléique,
g) la détection de la présence ou de l'absence d'un signal indiquant l'amplification du marqueur d'acide nucléique dans chacun de la pluralité de mélanges d'amplification individuellement et la détermination de la quantité de mélanges d'amplification présentant un signal positif, et
h) la comparaison de la quantité déterminée à l'étape g) avec une référence, en quantifiant ainsi la quantité de la molécule cible dans l'échantillon.

2. Procédé selon la revendication 1, dans lequel la molécule cible est
a) directement attachée au substrat solide, ou
b) attachée au substrat solide par une liaison à une molécule de capture qui est attachée au substrat solide.

3. Procédé selon la revendication 1 ou 2, dans lequel
a) le procédé comprend une étape de séparation entre l'étape b) et c) pour éliminer le conjugué de détection non lié, et/ou
b) la détection de l'amplification du marqueur d'acide nucléique à l'étape (g) comprend une détection par une sonde de détection.

4. Procédé selon l'une quelconque des revendications 1 à 3, dans lequel la liaison non covalente entre la molécule de liaison et le marqueur d'acide nucléique du conjugué de détection est facilitée par une interaction streptavidine/biotine ou avidine/biotine.

5. Procédé selon l'une quelconque des revendications 1 à 4, dans lequel les agents de libération comprennent une amorce d'acide nucléique qui est liée de façon covalente à l'agent de libération.

6. Procédé selon l'une quelconque des revendications 1 à 5, dans lequel l'amplification est
a) une réaction de PCR, ou
b) une réaction isotherme, facultativement sélectionnée dans le groupe consistant en une amplification basée sur une séquence d'acide nucléique (NASBA), une amplification médiée par la transcription (TMA), une amplification isotherme induite par boucle (LAMP), une amplification isotherme hélicase-dépendante (HDA), une amplification par recombinase et polymérase (RPA) et une amplification par déplacement de brin (SDA).

7. Procédé selon l'une quelconque des revendications 1 à 6, dans lequel le conjugué de détection comprend ou consiste en un anticorps biotinylé, une streptavidine (STV) tétravalente se liant à la biotine et un marqueur d'acide nucléique biotinylé.

8. Procédé selon l'une quelconque des revendications 1 à 7, dans lequel la molécule cible est un anticorps qui est attaché au substrat solide par l'interaction avec un antigène immobilisé sur le substrat solide et dans lequel la molécule de liaison du conjugué de détection est également un antigène dudit anticorps.

9. Procédé selon l'une quelconque des revendications 1 à 8, dans lequel la préparation de la pluralité de mélanges d'amplification est réalisée en utilisant
a) des gouttelettes/vésicules pour une encapsulation du mélange d'amplification ; ou
b) des microcavités.

10. Procédé selon l'une quelconque des revendications 1 à 9, dans lequel la liaison non covalente entre la molécule de liaison et l'acide nucléique est formée *in situ* au niveau de la molécule cible.

11. Utilisation d'une molécule de fusion comprenant une amorce d'acide nucléique et un agent de libération pour dissoudre la liaison non covalente d'un conjugué comprenant une molécule d'acide nucléique et une seconde molécule, dans laquelle l'agent de libération dissout la liaison non covalente par une liaison compétitive à au moins un membre de la liaison non covalente, où la liaison non covalente entre la molécule d'acide nucléique et la seconde molécule est formée par une interaction streptavidine/biotine ou avidine/biotine.

12. Kit comprenant
a) un conjugué de détection comprenant une molécule de liaison qui se lie spécifiquement à une molécule cible donnée et un marqueur d'acide nucléique, dans lequel la molécule de liaison et le marqueur d'acide nucléique sont liés par une liaison non covalente,
b) un réactif de dissolution qui dissout la liaison non covalente entre la molécule de liaison et le marqueur d'acide nucléique du conjugué de détection par une liaison compétitive à au moins un membre de la liaison non covalente, et
c) des agents d'amplification d'acide nucléique, dans lequel les agents d'amplification comprennent une amorce d'acide nucléique qui est attachée de façon covalente à l'agent de dissolution.
